# EUROPÄISCHE PATENTSCHRIFT

(11) **EP 1 938 825 B1**
(45) Veröffentlichungstag und Bekanntmachung des Hinweises auf die Patenterteilung: **25.06.2014**
(21) Anmeldenummer: 06450185.1
(22) Anmeldetag: 22.12.2006
(51) Int. Cl.: A61K 33/08

(54) **Einsatz von natürlichem Zeolith in der Therapie und Prophylaxe der Osteoporose**
Use of natural zeolite in the therapy and prevention of osteoporosis
Utilisation de la zéolithe naturelle pour le traitment et la prévention de la ostéoporose

(43) Veröffentlichungstag der Anmeldung: 02.07.2008
(73) Patentinhaber: Hraschan, Jakob, 9580 Drollobach (AT)
(72) Erfinder: Hraschan, Jakob, 9580 Drollobach (AT)
(74) Vertreter: Beckmann, Claus

(56) Entgegenhaltungen:
- EP-A1- 0 579 834
- EP-A1- 1 316 530
- EP-A2- 0 224 856
- WO-A-89/06965
- PAVELIC KRESIMIR ET AL: "Natural zeolite clinoptilolite: new adjuvant in anticancer therapy" JOURNAL OF MOLECULAR MEDICINE, SPRINGER VERLAG, DE, Bd. 78, 2001, Seiten 708-720, XP002191937 ISSN: 0946-2716
- POND W G ET AL: "BONE DENSITY AND TISSUE LEAD ACCRETION IN GROWING RATS FED LOW HIGH CALCIUM WITH OR WITHOUT SUPPLEMENTAL CLINOPTILOLITE." BULLETIN OF ENVIRONMENTAL CONTAMINATION AND TOXICOLOGY, SPRINGER VERLAG, NEW YORK, NY, US, Bd. 57, Nr. 5, November 1996 (1996-11), Seiten 713-721, XP008072344 ISSN: 0007-4861
- TRGO ET AL: "A comparative study of ion exchange kinetics in zinc/lead-modified zeolite-clinoptilolite systems" JOURNAL OF HAZARDOUS MATERIALS, ELSEVIER, Bd. 136, Nr. 3, 25. August 2006 (2006-08-25), Seiten 938-945, XP005594053 ISSN: 0304-3894

## Beschreibung

Die Erfindung bezieht sich auf Beeinflussung der Knochendichte und damit einer dadurch gegebenen Beeinflussbarkeit von Störungen im Aufbau des Knochengewebes, im engeren Sinne einer Verarmung an Kalzium oder auch Einbaustörungen von Kalzium im Knochengewebe bei Tier und Mensch. Das Hauptziel der Erfindung stellt hierbei insbesondere die Anwendung zur Therapie des weit verbreiteten Krankheitsbildes der Osteoporose und zur Verlangsamung des Prozesses der altersassoziierten Osteopenie dar.

In der WO 89/06965 A (entsprechend der EP 0 410 967 B), die einen guten Überblick über die Geschichte der oralen Verabreichung von Zeolithen an Mensch und Tier gibt, wird die orale Verabreichung von synthetischem Zeolith A in Verbindung mit einem Ansäuerungsmittel zur Beeinflussung kalziumabhängiger Störungen im Knochengewebe geoffenbart. Natürlicher Zeolith ohne Ansäuerungsmittel wird explizit als unwirksam bezeichnet.

Die Verwendung synthetischen Zeoliths A bietet zwar den Vorteil, sich nicht um Gewinnung und Reinigung des Zeoliths sorgen zu müssen, doch verliert man dadurch die auch in der WO-A vermutete Wirkung und Unterstützung der im natürlichen Zeolith enthaltenen Spurenelemente. Die Verwendung eines Ansäuerungsmittels ist für jeden Probanden belastend und stört den natürlichen Verdauungsvorgang, ist also insbesondere bei kranken oder älteren Probanden, somit der eigentlichen Zielgruppe, kontraproduktiv und stellt eine Kontraindikation dar.

Aus der EP 1 316 530 B, die zu einer ganzen Familie von Ausscheidungsanmeldungen gehört und auf der WO-0064586 A beruht, ist die Herstellung und Verwendung speziell gemahlenen Zeoliths zur Behandlung für eine ganze Reihe von Krankheiten, insbesondere Stoffwechsel und Herz-/Kreislauferkrankungen, multipler Sklerose, rheumatischen Erkrankungen und dermatologischen Erkrankungen bekannt.

In dem gegen dieses Patent erhobenen Einspruch wurde der Artikel "What the doctor should know about MEGAMIN and TMAZ!", veröffentlicht in "Medical News", Jahrgang 26, Nr. 141, November 1998, vorgelegt. in dem, ohne jede Quellenangabe oder nähere Details, in einem Nebensatz ausgeführt wird: " ... non-active zeolit ... offers protection against osteoporosis.". Dazu ist auszuführen, dass MEGAMIN der Handelsname des Produktes gemäß der EP 1 316 530 B ist und dass TMAZ für: "Tribo-Mechanisch-Aktivierter-Zeolith" steht. Dies bedeutet, dass sich das angegebene Zitat (non activated) auf Zeolith bezieht, der nicht gemäß der EP 1 316 530 B hergestellt worden ist. Diese Aussage steht somit im Gegensatz zur Aussage in der früheren, erstgenannten EP 0 410 967 B, darüberhinaus stehen beide Aussagen ohne jeden tatsächlichen, substantiierten Hintergrund für sich allein als reine Behauptung.

EP 0 224 856 A2 beschreibt eine Methode zur Verbesserung der Knochenqualität und/oder der Knochenstärke von Tieren, einschließlich Menschen, Rinder, Schafe, Ziegen, Schweine und Geflügel, ohne nachteilige Effekte auf diese Tiere oder Produkte dieser Tiere, wobei eine kleine effektive Menge Zeolithe entweder zu dem Futter der Tiere gegeben wird oder direkt, z.B. in Form einer Kapsel oder einer Tablette, den Tieren verabreicht wird.

Es hat sich bei Versuchen des Erfinders herausgestellt, das der gemäß der EP 1 316 530 B "aktivierte" Zeolith keine Wirksamkeit gegen Osteoporose bzw. altersassoziierter Osteopenie aufweist.

Es besteht somit Bedarf an einem zur Prophylaxe bzw. zur Behandlung von Osteoporose bzw. altersassoziierter Osteopenie geeigneten Medikament bzw. Wirkstoff.

Die Erfindung bezweckt, hier Abhilfe zu schaffen und hat das Ziel, einen Weg anzugeben, durch den natürlicher Zeolith ohne belastende Zugaben wirksam verabreicht werden kann. Doch vor der Darlegung der Erfindung soll deren medizinische und wirtschaftliche Bedeutung erläutert werden.

### Bedeutung der Erfindung

Im Kindes- und Jugendalter baut sich die Knochenmasse unter dem Einfluss von Sexualhormonen auf und erreicht um das 30. Lebensjahr den Höchstwert ("peak bone mass"). Nach dem 40. Lebensjahr kommt es bei allen Menschen zu einer langsamen Verminderung der Knochenmasse (physiologischerweise um 0,5 % jährlich). Dies bezeichnet man auch als altersassoziierten Knochenmassenverlust (Osteopenie).

Die langsame, aber stetig fortschreitende Demineralisierung der Knochen führt zu einer Rarefizierung der Spongiosa-Bälkchen und damit bei weiterem Fortschreiten zu einer verminderten Stützfunktion und Stabilität des Knochenapparates und geht bei stärkerer Ausprägung in der Folge mit einer gesteigerten Wahrscheinlichkeit von Frakturen einher. Besonders Frauen in der Postmenopause erwerben durch die veränderte hormonelle Situation einen zusätzlichen Risikofaktor für die Entstehung einer primären Osteoporose. In den ersten 10 Jahren nach der Menopause haben sie einen deutlich stärkeren Knochenverlust (2 % jährlich und mehr) als zuvor bzw. als Männer.

Aufgrund der osteoporotisch veränderten Knochenstruktur besteht bei ansonsten auch harmlosen Stürzen die Gefahr, typischerweise Schenkelhals-, Wirbel- oder Unterarmfrakturen zu erleiden.

Alleine in Österreich erleiden pro Jahr etwa 16 000 Personen einen Schenkelhalsbruch, wobei 35 Prozent der Patienten nach der operativen Versorgung innerhalb eines Jahres sterben. Die Behandlungskosten eines Patienten mit Oberschenkelhalsbruch von der Diagnose bis zur Gesundung belaufen sich auf rund 39.000 Euro.

Von der UNO und der WHO wurde die Osteoporose unter die 10 ökonomisch bedeutsamsten Volkskrankheiten des 21. Jahrhunderts eingestuft. In der Bundesrepublik beliefen sich die gesamten direkten Kosten der GKV für die Osteoporose (ICD-Nr. M80-M82) im Jahre 2002 gemäß den Auswertungen des Statistischen Bundesamtes 2004 auf 1.399 Millionen Euro.

### Die Erfindung

Die Erfindung löst die oben gestellte Aufgabe dadurch, dass natürlicher Zeolith , der eine Korngrößenverteilung mit einem Maximum bei etwa 5 Mikrometer Korngröße oder bis 15% weniger aufweist, wobei 90 Gewichts-% des Zeoliths eine Korngröße zwischen 0,8 Mikrometer und 40 Mikrometer aufweisen und bevorzugt 80 Gewichts-% eine Korngröße zwischen 1 Mikrometer und 30 Mikrometer aufweisen, zur Herstellung von pharmazeutischen Zubereitungen für die Behandlung, Vorbeugung oder Verzögerung des Krankheitsbildes der Osteoporose und zur Verlangsamung des Prozesses der altersassoziierten Osteopenie in menschlichen Lebewesen verwendet wird. Es hat sich herausgestellt, dass bei dieser Korngröße bzw. Korngrößenverteilung eine bisher weder beobachtete noch erwartete Wirksamkeit eintritt. Vermutlich wurde bei der Erforschung der Wirksamkeit der Zeolithe in den letzten Jahren allzu großes Augenmerk auf immer kleinere Korngrößen und die damit zu erzielenden Effekte gelegt.

Als Korngröße ist bei merklichen Abweichungen der Gestalt des Kornes von der Kugelgestalt der Durchmesser einer Kugel mit gleichem Volumen wie das Korn anzusetzen.

Eine solche Korngrößenverteilung und Korngröße kann bevorzugt durch Zerkleinern des Ausgangsmaterials zwischen zwei gegensinnig laufenden Rotoren, die aus Scheiben bestehen, auf denen mit Kränzen verbundene Ventilatorschaufeln angeordnet sind, erfolgen, wobei die Ventilatorschaufeln jeweils in korrespondierende Kanäle zwischen den Ventilatorscheiben der gegenüberliegenden Rotorenscheibe durchgreifen, wobei das Ausgangsmaterial durch ein Einsaugrohr in den Zentralteil der rotierenden Scheibe eingebracht wird und aufgrund der Zentrifugalkräfte die fortlaufend zerkleinerten Körnchen des Ausgangsmaterials in Richtung des äußeren Randes des Gehäuses beschleunigt und dort als Endmaterial entnommen werden.

Die Verabreichung erfolgt ohne gleichzeitige Gabe von Ansäuerungsmitteln oder anderen, den Organismus bzw. Metabolismus der Probanden belastenden Zusätzen, wenn auch bevorzugt mit anderen aus dem Stand der Technik stammenden protektiven Substanzen und stellt so gegenüber dem im Stand der Technik verwendeten synthetischen Zeolith A mit Ansäuerungsmittel eine deutliche Verbesserung und Erleichterung für den Probanden dar.

Die Verabreichung des Zeoliths, bevorzugt in Verbindung mit den bereits bekannten protektiven Faktoren wie ausreichende körperliche Aktivität (diese stellt den physiologischen Reiz für die Knochenbildung und damit die Basis für jede Prävention und Therapie von Osteoporose dar) sowie eine Ernährung mit ausreichender Zufuhr von Kalzium und Vitamin D ist die Grundlage für Prophylaxe und Therapie. Der beschriebene, oral verabreichte Zeolith ist aufgrund seiner Ionenaustauschkapazität in der Lage, Kalzium in anscheinend sehr gut bioverfüglicher Form abzugeben. Außerdem scheint der Zeolith auch geringe Anteile des in ihm enthaltenen Siliziums in kolloidaler Form abzugeben, dies dürfte sich zusätzlich positiv auf die Knochendichte auswirken.

So erweitert der Zeolith das bisherige Spektrum der Prophylaxe, das sich bislang aus kalziumreicher Diät, Steigerung der körperlichen Aktivität und bei postmenopausaler Osteoporose in der Gabe von Östrogenen bestand. Es wäre ein beträchtlicher und nunmehr durchaus im Bereich des Möglichen liegender Fortschritt, könnte die Osteoporoseprophylaxe dadurch überhaupt auf die Östrogentherapie verzichten, da extern zugeführte Hormone eine Irritation für die fein abgestimmten Regelkreise des Organismus darstellen und die Östrogengabe ein erhöhtes Risiko der Entwicklung eines Endometriumkarzinoms in sich birgt.

Auch zeigt sich am vorliegenden Fall, dass sich die Wirkung der oralen Verabreichung von tribomechanisch mikronisiertem Zeolith keinesfalls auf die Prophylaxe beschränkt, sondern auch bei bereits manifester Osteoporose eine Verbesserung der Knochendichte bewirkt und damit eine sehr wirksame therapeutische Maßnahme darstellt, welche die übrigen therapeutischen Maßnahmen (wie Calcitonin, Bisphosphonate oder Fluoride) ergänzen oder im individuellen Fall auch ersetzen kann.

### Hintergründe der Erfindung

Zugrunde liegt ein gut dokumentierter Fall einer im Jahre 1929 geborenen Patientin, bei der am 24.11.1999 bei der Knochendichtemessung mittels DEXA (dual-energy x-ray absorptiometry) mit dem Gerät HOLOGIC QDR-1000 im Oberschenkelhals (femoral neck) ein T-Score von -2,99 (67 % des geschlechtsspezifischen Sollwerts entsprechend), im distalen Radius wurde ein T-Score von -3,5 gemessen. Anhand des Befundes musste die Diagnose "Osteoporose" gestellt werden, da laut WHO ein kleinerer T-Score als - 2,5 definitionsgemäß als Osteoporose interpretiert wird.

Im Befundbericht vom 3.12.1999 wurde ein medikamentöser Therapievorschlag von Fosamax 10 mg (ein Bisphosphonat) 1x täglich morgens und täglich ½ Beutel Maxikalz-Vitamin D3 gemacht. Diese beiden Medikamente wurden von der Patientin jedoch nur kurze Zeit eingenommen und dann wegen Unverträglichkeit abgesetzt.

Anstelle dessen begann die Patientin im Januar 2000 mit der Einnahme des unten genauer beschriebenen, gemahlenem Zeolithen, den sie vom Erfinder und Anmelder erhalten hatte. Eine Kapsel des Produkts enthielt 750 mg des gemahlenen Zeolithen. Von dieser Substanz wurde von der Patientin eine Tagesdosis zwischen 4500 mg und 6000 mg eingenommen. Der Zeolith wird von der Patientin bis zum heutigen Tag eingenommen, wobei sich die Schwankungen der Tagesdosen aus der Veränderung der Dosierungsempfehlungen im Laufe der Jahre ergaben.

Die Kontrolle erfolgte am 25.4.2001 mit demselben Gerät in derselben osteologischen Ambulanz, in welcher der Erstbefund erstellt wurde: Bei dieser ergab sich ein T-Score des Oberschenkelhalses (femoral neck) von -1,92 (79 % des geschlechtsspezifischen Sollwerts), die Bestimmung erfolgte mit derselben Methode sowie demselben Gerät, und stellt damit eine außerordentliche Verbesserung der Knochendichte im Vergleich zum Erstbefund dar. Definitionsgemäß kann man ab diesem Zeitpunkt also nicht mehr von Osteoporose sprechen (auch wenn die Diagnose im entsprechenden Arztbrief fälschlicherweise weitergeführt wird), sondern es handelt sich hier um den Prozess der altersassoziierten Osteopenie. Es wurde sowohl bei der Erstuntersuchung als auch bei der Kontrolle die linke Hüfte vermessen, sodass die Entstehung eines Fehlers durch die Bestimmung der kontralateralen Seite nicht in Frage kommt.

Bei einer weiteren Osteodensitometrie am 21.7.2005 ergab sich, diesmal mit dem Gerät DEXA QDR-4500 C gemessen, ein T-Score von -1,4 (femoral neck) und somit eine weitere Verbesserung der Knochendichte des linken Oberschenkelhalses. Dadurch ist eine Verifizierung des Vorbefundes gegeben und eine Nachhaltigkeit des Ergebnisses dokumentiert.

Die Ergebnisse sind auch nicht durch eine gesteigerte körperliche Aktivität ab dem Zeitpunkt der Diagnose erklärbar, da die Patientin schon davor regelmäßig Gymnastik betrieben hatte und da der Lebensstil, was die körperliche Aktivität und auch sonstige Bereiche anbelangt, nach Erhebung des Erstbefundes nicht wesentlich verändert wurde.

### Vermutete Wirkmechanismen

Die Fähigkeit des Zeoliths, eine manifeste Osteoporose rückgängig zu machen, dürfte sich aus mehreren verschiedenen Mechanismen zusammensetzen:
Einen wesentlichen Beitrag leistet sicherlich die Ionenaustauschfähigkeit des Zeoliths. Im Gastrointestinaltrakt werden aus der Eiweißverdauung entstehende Ammoniumionen, Schwermetallionen, Übergangsmetallionen und auch eventuell vorhandene Radioisotope (Cd²⁺, NH₄⁺, Hg²⁺, Fe³⁺, Pb²⁺, Cu²⁺, Cs⁺, Sr²⁺), zu denen der Zeolith eine höhere Affinität hat, gegen die im Zeolith eingelagerten freien Kationen (Na⁺, K⁺, Ca²⁺, Mg²⁺) ausgetauscht. Entscheidend für die vorliegende Erfindung ist dabei die Abgabe von anscheinend sehr gut bioverfügbaren Kalziumionen, die in Form von Hydroxylapatit in den Knochen eingebaut werden können. Es dürfte sowohl zu einer besseren Resorption des im Verbund mit dem Zeolithen dargereichten Kalziums als auch zu einer erhöhten Einbaurate des resorbierten Kalziums im Rahmen der Mineralisation des Knochens kommen.

Es ist hinlänglich bekannt und vielfach belegt, dass Zeolith in der Lage ist, als Ionenaustauscher zu fungieren und in vitro Ca²⁺-Ionen abzugeben. Einen Beweis dafür, dass die orale Verabreichung von Zeolith auch in vivo den Kalziumhaushalt nachhaltig beeinflussen kann, liefert eine Studie , in der die Auswirkungen der Nahrungsergänzung mit Klinoptilolith auf die Inzidenz des Milchfiebers bei 52 Milchkühen untersucht wurde. Milchfieber (auch Gebärparese, Kalbefieber oder engl. parturient paresis) ist eine Krankheit bei Säugern, die um den Geburtszeitpunkt der Frucht auftreten kann und durch einen verminderten Kalziumgehalt des Blutserums (Hypokalzämie) verursacht wird. Diese Erkrankung stellt eine der häufigsten Erkrankungen der Milchviehwirtschaft dar. Typischer Zeitpunkt des Auftretens ist unmittelbar nach der Geburt bis ca. 2 Tage danach, da es hier zu einer Umstellung der Stoffwechsellage kommt, in der bei der Laktation mit der Milch eine beträchtliche Menge an Kalzium abgeführt wird. Nach Beginn der Symptomatik mit taumelndem Gang und in der weiteren Folge dem Unvermögen des Aufstehens kommen eine erhöhte Pulsfrequenz (Tachykardie) und entgegengesetzt der Krankheitsbezeichnung ein Absinken der Körpertemperatur (Hypothermie) hinzu. Mit fortschreitender Dauer trübt sich das Bewusstsein ein, die Muskelkontraktionen und die Herzfrequenz sinken ab, so dass es ohne Behandlung bei ca. 75 % der Tiere zum Tod kommt. Die gängige Therapie besteht in der Infusion stark kalziumhaltiger Lösungen, auch zur Vorbeugung hat sich die orale Gabe von Kalziumpräparaten unmittelbar nach der Geburt bewährt.

Die oben erwähnte Studie ergab bei 2,5 %-iger Klinoptilolith Beimengung zum Futter eine signifikant niedrigere Inzidenz der Erkrankungen an Milchfieber und stellt somit einerseits eine kostengünstige Form der Prävention der Erkrankung dar, andererseits wird damit eine positive Beeinflussung des Kalziumhaushalts durch orale Verabreichung von Zeolith nahe gelegt, da ja die Hypokalzämie die Ursache der Erkrankung ist. Diese positive Beeinflussung des Kalziumhaushalts spielt höchstwahrscheinlich auch bei der vorliegenden Erfindung die bedeutendste Rolle.

Bezüglich der Osteoporose scheint außerdem auch die bereits in vitro und in vivo nachgewiesene antioxidative Aktivität des Zeoliths mit der daraus resultierenden Fähigkeit zur Reduktion von oxidativem Stress eine Rolle zu spielen. Durch oxidativen Stress werden nämlich Pathomechanismen in Gang gesetzt, die in Zusammenhang mit einer Vielzahl von degenerativen Erkrankungen. Hier steht vor allem die Arteriosklerose mit ihren Folgeerkrankungen Herzinfarkt, Schlaganfall und periphere arterielle Verschlusskrankheit im Vordergrund. Gesundes Knochengewebe ist am lebenden Menschen nicht tot, sondern es finden darin ständig funktionsangepasste Knochenumbauprozesse statt (bestehend aus Knochenaufbau durch Osteoblasten und Knochenresorption durch Osteoklasten). Um die Versorgung mit den dafür nötigen "Baustoffen" zu gewährleisten, ist eine einwandfreie Durchblutung des Knochens die Voraussetzung. Zweifelsohne ist auch die Osteoporose eine degenerative Erkrankung, zu deren Entstehung auch die unzureichende Versorgung mit Nährstoffen aufgrund arteriosklerotisch veränderter Blutgefäße beiträgt.

Da die Entstehung der Arteriosklerose nachweislich durch reaktive Sauerstoffspezies mitbedingt ist und einen lebenslangen Prozess darstellt, greift der oral verabreichte Zeolith auch auf dieser Ebene ins Geschehen ein, da er imstande ist, trotz seines Wirkortes im Magen-Darmtrakt die Konzentration von reaktiven Sauerstoffspezies im Blut zu vermindern und damit höchstwahrscheinlich auch die Gefäßwandschädigungen zu verzögern.

### Zeolith - ein kurzer Überblick

Bereits seit Jahrzehnten finden das vulkanische Mineral Zeolith in der Industrie und Forschung wegen seiner Adsorptionseigenschaften und Fähigkeit zum Ionenaustausch zahlreiche Anwendungen, sei es zur Trink- und Abwasserreinigung, zur Wasserentkalkung, in der Medizin als Hämodialysefilter oder bei der Bunt- und Schwermetallgewinnung.

Der Zeolith ist ein natürliches mikroporöses Gestein vulkanischen Ursprungs. Chemisch gesehen handelt es sich dabei um ein hydratisiertes Alumosilikat der alkalischen Metalle und der Metalle von alkalischen Erden. Der Name "Zeolith" leitet sich aus dem Griechischen von "zeo" = "ich siede" und "lithos" = "Stein" her, bedeutet also "Siedestein". Dies hat damit zu tun, dass Zeolithe beim Erhitzen ohne einen Zerfall des Aluminosilikatgerüsts gebundenes Wasser abgeben, sie "sieden".

Die Kristallgitter der Zeolithe bestehen aus SiO₄- und AlO₄-Tetraedern, die über Sauerstoff-Brücken miteinander verbunden sind. Dabei entsteht eine komplexe räumliche Anordnung regelmäßiger Hohlräume mit verschiedenen, genau definierten Porengrößen (ca. 4 Angström, entsprechend 0,4 nm). In diesen Kristallgittern befinden sich Kationen wie Kalzium, Magnesium, Natrium, Kalium u.a. im Verbund mit Kristallwasser. Dieser Aufbau der Zeolithe ergibt eine außerordentlich große spezifische Oberfläche von ca. 1000 Quadratmetern pro Gramm Zeolith.

Es gibt mehr als 100 verschiedene Zeolitharten, die in Faserzeolithe, lamellare Zeolithe und Würfelzeolithe eingeteilt werden können. Für die Anwendung im Bereich der Human-und Veterinärmedizin hat sich der Klinoptilolith, ein lamellarer Zeolith, mit seinen herausragenden Eigenschaften im Laufe der Zeit als am geeignetsten herausgestellt. Zeolith ist in Japan bereits seit 1996 als Lebensmittelzusatzstoff zugelassen, in der Humanmedizin wurden seit 1986, soweit dem Anmelder bekannt, weltweit 39 auf Zeolith Bezug nehmende Patente angemeldet.

Die orale Verabreichung an Tier und Mensch setzt gewisse Ansprüche voraus, die der verwendete Zeolith zu erfüllen hat: Er ist stabil gegenüber Säuren und Laugen, ist thermisch stabil bis 450 °C, ist nicht toxisch, weiters gibt es keine gefährliche Zersetzung oder Polymerisierung und ist außerdem nicht wasserlöslich. Die fehlende Wasserlöslichkeit ergibt, dass der beschriebene Zeolith nicht als solcher aus dem Gastrointestinaltrakt resorbiert wird, jedoch kann er hier seine Wirkung als Adsorbens und Ionenaustauscher entfalten und Ca-Ionen in anscheinend sehr gut bioverfügbarer Form abgeben.

Bei der oralen Verabreichung von Zeolithen stehen zwei Hauptfunktionen im Vordergrund:
a) Ionenaustausch:
   Zeolithe können ihre freien Kationen (Na⁺, K⁺, Ca²⁺, Mg²⁺) gegen Schwermetalle, Ammoniumionen, Radioisotope oder andere Kationen (Cd²⁺, NH₄⁺, Hg²⁺, Fe³⁺, Pb²⁺, Cu²⁺, Cs⁺, Sr²⁺) austauschen, für welche eine größere Selektivität besteht. Dies ist eine entscheidende Fähigkeit des Klinoptilolith.
b) Adsorption:
   Zeolithe adsorbieren niedrigmolekulare Verbindungen (wie Kohlenwasserstoffe, Schwefeldioxid und Stickoxide) und auch Mycotoxine. Sie wirken als Molekularsieb und absorbieren Gase und gelöste Stoffe bestimmter Größe.

### Daten des im speziellen Fall verwendeten Zeolithen:

| **Mineralogische Zusammensetzung (Gew-%, typisch)** | |
|---|---|
| • Klinoptilolith | 84 % |
| • Cristobalit | 8 % |
| • Feldspat | 4 % |
| • lllit | 4 % |
| • Quarz | Spuren |
| • Karbonatminerale | < 0.5 % |
| Tabelle 1 | |

| **Chemische ZusammenSetzung (Gew-%)** | |
|---|---|
| SiO₂ | 65,0 - 71,3 % |
| Al₂O₃ | 11,5 - 13,1 % |
| CaO | 2,7 - 5,2 % |
| K₂O | 2,2 - 3,4 % |
| Fe₂O₃ | 0,7 - 1,9 % |
| MgO | 0,6 - 1,2 % |
| Na₂O | 0,2 - 1,3 % |
| TiO₂ | 0,1 - 0,3 % |
| Tabelle 2 | |

| **Gesamtaustausch**: | |
|---|---|
| Ca²⁺ | 0,64 - 0,98 mol/kg |
| Mg²⁺ | 0,06 - 0,19 mol/kg |
| K⁺ | 0,22 - 0,45 mol/kg |
| Na⁺ | 0,01 - 0,19 mol/kg |
| NH₄⁺ | 1,2 - 1,5 mol/kg |
| Tabelle 3 | |

| **Wasserdampfsorption durch dehydratisiertes Gestein:** |
|---|
| • bei 20 °C und 52 % rel. Feuchte: 7,5 - 8,5 g H₂O/100g |
| • bei 20 °C und 98 % rel. Feuchte: 13,5-14,5 g H₂O/100g |
| Tabelle 4 |

Die Erfindung ist nicht auf das beschriebene Beispiel beschränkt, sondern kann verschiedentlich abgeändert und variiert werden. So ist es möglich, im Rahmen der genannten Kriterien einen anderen natürlichen Zeolithen zu verwenden, die Zerkleinerung des Rohmaterials kann auf andere als die angegebene Weise erfolgen und die tägliche Dosierung ist in weiten Grenzen an das Alter, das Krankheitsbild und den Allgemeinzustand des Probanden bzw. Patienten anpassbar. Es können alle derzeit bekannten und sicherlich auch die in Zukunft entwickelten, den Knochenaufbau fördernden und/oder unterstützenden Medikamente mit dem erfindungsgemäßen Zeolith verabreicht werden.

## Patentansprüche

1. Verwendung von natürlichem, zerkleinertem Zeolith mit einer Korngrößenverteilung mit einem Maximum bei etwa 5 Mikrometer Korngröße oder bis 15% weniger, wobei 90 Gewichts-% des Zeoliths eine Korngröße zwischen 0,8 Mikrometer und 40 Mikrometer aufweisen und bevorzugt 80 Gewichts-% eine Korngröße zwischen 1 Mikrometer und 30 Mikrometer aufweisen, zur Herstellung von pharmazeutischen Zubereitungen für die Behandlung, Vorbeugung oder Verzögerung des Krankheitbildes der Osteoporose und zur Verlangsamung des Prozesses der altersassoziierten Osteopenie in menschlichen Lebewesen.

2. Verwendung gemäß Anspruch 1, wobei die pharmazeutische Zubereitung den Zeolith in einem Anteil von 50 Gewichts-% bis 100 Gewichts-% enthält.

3. Verwendung gemäß Anspruch 2, wobei die pharmazeutische Zubereitung oral in einer solchen Menge verabreicht wird, dass die tägliche Zufuhr an Zeolith zwischen 3000 mg und 8000 mg, bevorzugt zwischen 4500 mg und 6000 mg ausmacht.

4. Verwendung gemäß Anspruch 2 oder 3, wobei die pharmazeutische Zubereitung oral in einer solchen Menge verabreicht wird, dass die tägliche Zufuhr an Zeolith zwischen 70 mg/kg Körpergewicht und 140 mg/kg Körpergewicht ausmacht.

## Claims

1. The use of natural, comminuted zeolite having a grain size distribution with a maximum at about 5 µm grain size or up to 15 % less, 90 wt.-% of the zeolite having a grain size between 0.8 µm and 40 µm, and preferably 80 wt.-% having a grain size between 1 µm and 30 µm, for the preparation of pharmaceutical preparations for the treatment, prevention or delay of the symptoms of osteoporosis and for slowing down the process of age-associated osteopenia in humans.

2. The use according to claim 1, where the pharmaceutical preparation contains the zeolite at a ratio of 50 wt.-% to 100 wt.-%.

3. The use according to claim 2, where the pharmaceutical preparation is administered orally in such an amount that the daily supply of the zeolite is between 3,000 mg and 8,000 mg, preferably between 4,500 and 6,000 mg.

4. The use according to claim 2 or 3, where the pharmaceutical preparation is administered orally in such an amount that the daily supply of the zeolite amounts to between 70 mg/kg body weight and 140 mg/kg body weight.

## Revendications

1. Utilisation d'une zéolithe naturelle broyée ayant une distribution des tailles de grains avec un maximum à une taille de grain d'environ 5 micromètre ou jusqu'à 15% moins, dans laquelle 90% en poids de la zéolithe ont une taille de grain comprise entre 0,8 micromètre et 40 micromètre, et de préférence 80% en poids ont une taille de grain comprise entre 1 micromètre et 30 micromètre, pour la fabrication des préparations pharmaceutiques pour le traitement, la prévention ou le retardement des signes cliniques de l'ostéoporose et pour le ralentissement du procès de l'ostéopénie due à l'âge chez des êtres humains.

2. Utilisation selon la revendication 1, dans laquelle la préparation pharmaceutique contient de la zéolithe en une quantité de 50% en poids à 100% en poids.

3. Utilisation selon la revendication 2, dans laquelle la préparation pharmaceutique est administrée par voie orale dans une quantité telle que l'apport journalier de la zéolithe est compris entre 3000 mg et 8000 mg, de préférence entre 4500 mg et 6000 mg.

4. Utilisation selon la revendication 2 ou 3, dans laquelle la préparation pharmaceutique est administrée par voie orale en une quantité telle que l'apport journalier de la zéolithe est compris entre 70 mg/kg de poids corporel et 140 mg/kg de poids corporel.
